# EUROPEAN PATENT SPECIFICATION

(11) **EP 4 245 856 B1**
(45) Date of publication and mention of the grant of the patent: **30.10.2024**
(21) Application number: 22162240.0
(22) Date of filing: 15.03.2022
(51) Int. Cl.: C12P 21/02, C12N 9/08

(54) **SYNTHESIS OF FUNCTIONAL UNSPECIFIC PEROXIDASES USING A NON-FUNGAL EUKARYOTIC CELL-FREE SYSTEM**
SYNTHESE FUNKTIONELLER UNSPEZIFISCHER PEROXIDASEN UNTER VERWENDUNG EINES NICHT-FUNGALEN EUKARYOTISCHEN ZELLFREIEN SYSTEMS
SYNTHÈSE DE PEROXYDASES FONCTIONNELLES NON SPÉCIFIQUES À L'AIDE D'UN SYSTÈME EUCARYOTE ACELLULAIRE NON FONGIQUE

(43) Date of publication of application: 20.09.2023
(73) Proprietor: Fraunhofer-Gesellschaft zur Förderung der angewandten Forschung e.V., 80686 München (DE)
(72) Inventor: WALTER, Ruben, 14476 Potsdam (DE); KUBICK, Stefan, 14476 Potsdam (DE); ZEMELLA, Anne, 14476 Potsdam (DE)
(74) Representative: v. Bezold & Partner Patentanwälte - PartG mbB

(56) References cited:
- SCHRAMM MARINA ET AL: "Cell-Free Protein Synthesis with Fungal Lysates for the Rapid Production of Unspecific Peroxygenases", ANTIOXIDANTS, vol. 11, no. 2, 30 January 2022 (2022-01-30), pages 284, XP055952470, DOI: 10.3390/antiox11020284
- ANNE ZEMELLA ET AL: "Cell-Free Protein Synthesis: Pros and Cons of Prokaryotic and Eukaryotic Systems", CHEMBIOCHEM, vol. 16, no. 17, 19 October 2015 (2015-10-19), pages 2420 - 2431, XP055752493, ISSN: 1439-4227, DOI: 10.1002/cbic.201500340
- ANDREAS K BRÖDEL ET AL: "Cell-free protein expression based on extracts from CHO cells", BIOTECHNOLOGY AND BIOENGINEERING, JOHN WILEY, HOBOKEN, USA, vol. 111, no. 1, 6 September 2013 (2013-09-06), pages 25 - 36, XP071145973, ISSN: 0006-3592, DOI: 10.1002/BIT.25013
- ANGELO C BATISTA ET AL: "Optimising protein synthesis in cell-free systems, a review", ENGINEERING BIOLOGY, JOHN WILEY & SONS, INC, HOBOKEN, USA, vol. 5, no. 1, 21 February 2021 (2021-02-21), pages 10 - 19, XP006113745, ISSN: 2398-6182, DOI: 10.1049/ENB2.12004

## Description

### Background of the invention

Unspecific peroxygenases (UPOs) are a group of fungal enzymes (EC 1112.1), carrying a cysteinate ligated heme as prosthetic group, critical for its catalytic function. UPOs catalyze a variety of oxyfunctionalization reactions and UPOs as an enzyme class show a broad range of substrates, ranging from small aromatic molecules over fatty acids to steric demanding substrates like testosterone. Due to the broad range of substrates and catalyzed reactions UPOs are promising candidates for the development of new biosynthetic pathways, not only for the generation of renewable platform chemicals but also for generating molecules of pharmacological importance. In contrast to the cytochrome P450 monooxygenases, that show similar catalytic properties, UPOs are not dependent on coenzymes or expensive cofactors, demanding only H₂O₂. Thus, UPOs can be regarded as self-sufficient biocatalysts (e.g., Hofrichter und Ullrich (2006) in Applied microbiology and biotechnology 71 (3), 276-288).

Since their first description (Ullrich et al. (2004) in Applied and environmental microbiology 70 (8), 4575-4581), more than 4800 putative UPOs from more than 2500 fungal genomes were predicted. However, until today only about 20 different UPOs and variants thereof were successfully expressed and characterized (Püllmann et al. (2021) in Communications biology 4 (1), 562, and Hofrichter et al. (2022) in Antioxidants (Basel, Switzerland) 11 (1)). First approaches utilized native UPO-producing hosts for expression (e.g., Ullrich et al. 2004; Ullrich et al. (2018) in Journal of inorganic biochemistry 183, 84-93; Kinner et al. (2021) in Frontiers in bioengineering and biotechnology 9, 705630). Though this approach is able to achieve high yields, the cultivation is time consuming, taking up to several weeks (Ullrich et al. 2018). Since medium composition and cultivation conditions are critical for UPO production (Hofrichter et al. (2015) in Advances in experimental medicine and biology 851, 341-368), the optimization of UPO production is a timely process. Furthermore, the native UPO hosts have to be cultivatable under laboratory conditions in the first place. Even for those hosts that can be cultivated, the lack of established genetic tools renders them less suitable for functional studies and enzyme engineering approaches.

Utilizing classical fungal production hosts like *Saccharomyces cerevisiae* (Molina-Espeja et al. (2014) in Applied and environmental microbiology 80 (11), 3496-3507; Püllmann et al. (2021) above, Pichia pastoris (e.g., Molina-Espeja et al. (2015) in Enzyme and microbial technology 73-74, 28-33; Püllmann et al. (2021)), Aspergillus oryzea (Babot et al. (2013) in Biotechnology and bioengineering 110 (9), 2323-2332) and *Aspergillus niger* (Conesa et al. (2001) in The Journal of biological chemistry 276 (21), 17635-17640), 13 different UPOs were expressed. Of those 8 UPOs were not isolated before. The expression of UPOs in known biotechnological hosts, opened the possibilities for extensive engineering and directed evolution approaches to improve catalytic properties (e.g. Molina-Espeja et al. 2015). This underlines the importance for the expression system to allow for parallel assessment of multiple UPO variants.

While expression of recombinant UPOs infungal hosts proved valuable in some cases, the transfer of this methodology to other UPOs is not trivial. Extensive optimization and engineering of the signal peptide might be required to achieve efficient expression and secretion of the UPOs of interest (Molina-Espeja et al. 2014; Püllmann et al. 2021). Expression of UPOs in *Escherichia coli* is currently only reported for three UPOs, with limited yields of purified, functional enzyme (Carro et al. (2019) in ACS Catal. 9 (7), 6234-6242; Linde et al. ( 2020) in Applied and environmental microbiology 86 (7); Fernandez Fueyo et al. (2019) in WO/2020011847 A1), presumably due to the lack of PTMs and chaperones (Kinner et al. 2021).

A further approach for the production of recombinant UPOs was just recently reported, employing cell-free protein synthesis (CFPS) based on fungal cell lysates (Schramm et al. (2022) in Antioxidants 11 (2), 284). In CFPS, translationally active cell-lysate is utilized to perform translation reactions. Due to its open nature reaction, conditions can be directly manipulated, making it a versatile tool for fast protein production.

In this CFPS approach, the use of fungal cell lysates appears to be a first choice due to the phylogenetic proximity to UPO producers (in fact both host systems used in Schramm et al., ibid, do produce UPOs themselves). However, as may be concluded from the results obtained in classical systems for protein synthesis utilizing various fungal hosts for UPO expression, likely fungal based CFPS systems are also only applicable to some UPOs. Here, also extensive optimization and engineering of the signal peptide might be required to achieve efficient expression and translocation of the UPOs of interest. Moreover, the full capacity of synthesis of the *Neurospora crassa* based CFPS-system used in Schramm et al. remains uncertain, making it difficult to assess its ability to synthesize sufficient amounts of UPO other than AaeUPO in order to detect functionality.

Further, Schramm et al. only describe a cell-free system utilizing linked CFPS (mRNA as template) and not coupled CFPS (DNA as template).

Especially, when it comes to screening, coupled CFPS is much more flexible and highthroughput suitable. This holds especially true when using PCR-products as templates. One drawback of this approach, however, is their stability. This problem has been successfully addressed for CFPS systems based on insect and mammalian cell lysates, whereas it remains unclear to what extent stability of linear DNA has to be addressed in fungal systems.

On the other hand, the transfer of this fungal CFPS approach to other non-fungal based CFPS systems is not trivial as well. The exact biogenesis of UPOs and involved chaperones remains elusive. Essential chaperones and other proteins may be involved in folding and heme integration might not be present in non-fungal systems. Further, UPOs display a variety of N-terminal peptides, that are processed during secretion/maturation (Hofrichter 2022) that in part greatly differ from the signal peptides utilized in the described insect-based system. Because of their structural diversity and their uncertain mechanistic involvement in protein export, these N-terminal peptides in their entirety are hereafter generally referred to as signal peptides. The signal peptides not only need to mediate efficient translocation in the host system, but also have to be processed in a manner, that preserves the native N-terminus of the processed UPO. Alteration at the N-terminus can affect enzyme activity dramatically. Therefore, it cannot be assumed that the wild-type signal peptides will drive translocation in insect-based CFPS.

For insect-based CFPS the utilization of an exogenous signal peptide, i.e. the melittin signal peptide, has proven suitable for the translocation of different proteins (e.g., Sonnabend et al. (2017) in *Biotechnology and bioengineering* 114 (10). However, a prerequisite for the utilization of an exogenous signal peptide in the CFPS synthesis of UPOs is the correct identification and replacement of the respective native fungal signal peptide by said exogenous signal peptide, which is not trivial as well.

Summarizing, presently there is still no universally applicable production platform available for UPOs, which would allow the screening for new UPO and the efficient engineering of known UPOs. Existing efficient production pipelines are limited to a small number of UPOs, and while great progress was made in the past two decades, the expression and characterization of new UPOs still poses a time consuming challenge.

In view of this situation, the main object underlying the present invention is the provision of novel and improved means for cell-free synthesis of a broad range of functional unspecific peroxygenases, which overcome or considerable alleviate the drawbacks of the prior art.

This object is achieved according to the present invention by providing the method for preparing a functional unspecific peroxygenase (UPO) using a cell-free eukaryotic system based on insect cells or mammalian cells according to claim 1 as well as the reaction medium for use in such a method according to claim 12. Additional aspects and preferred embodiments of the present invention are the subject of further claims.

### Description of the invention

The method of the present invention for preparing a soluble and functional peroxygenase in cell-free systems comprises at least the following steps:
a) providing a reaction medium comprising at least the following components:
   - a nucleic acid template coding for a fungal unspecific peroxygenase (UPO) classified as EC 111.2.1, an RNApolymerase, a eukaryotic cell lysate derived from insect cells or mammalian cells and supplemented with glutathione and glutathione-disulfide during lysate preparation wherein the cell lysate further comprises non-fungal membrane vesicles, as well as heme or heme derivatives, in particular heme complexes selected from the group comprising or consisting of hemin and hematin;
b) performing a cell-free reaction to synthesize the peroxygenase protein.

More specifically, the reaction medium comprises the heme or heme derivative, in particular hemin or hematin, in a concentration in the range of from 1 µM to 100 µM, preferably from 15 µM to 20 µM.

In another preferred embodiment, the cell-free translation reaction is effected at a temperature in the range from 21 to 33 °C.

The nucleic acid template coding for a fungal unspecific peroxygenase may be a mRNA template or a DNA template and preferably is a DNA template.

Particularly in insect cell-based systems, optimized workflows for linked CFPS (mRNA as template, as performed in Schramm et al.) as well as coupled CFPS (DNA as template) have been established (e.g. Kubick et al. (2009) in Current topics in membranes, 63, 25-49).

Preferably, the nucleic acid template further comprises a nucleic sequence coding for a melittin signal peptide.

For insect-based CFPS the utilization of the melittin signal peptide has proven suitable for the translocation of different proteins (e.g., Sonnabend et al. (2017), Stech et al. (2013). Besides its beneficial effect on translocation in insect and mammalian CFPS, the melittin signal peptide also increases translation efficiency (Brödel et al. 2014), making the melittin signal peptide far more efficient than most native signal peptides, in term of mammalian and insect-based CFPS.

However, a prerequisite for the utilization of the melittin signal peptide is the correct identification and replacement of the native signal peptide by said melittin signal peptide. As shown by the present inventors, this has been achieved and the melittin signal peptide proved itself to be suitable for the efficient translocation, resulting in active UPO, even for AaeUPO, which is known to have a rather low promiscuity towards signal peptides (Püllmann 2021). A signal peptide, with similar universality like melittin is yet to be found for fungal CFPS.

Advantageously, the cell-free preparation method according to the present invention is applicable to broad range of unspecific peroxygenases (UPOs) including long-chain UPOs and short-chain UPOs.

Typically, the long-chain unspecific peroxidase comprises a sequence of amino acids in the range from 300 to 400 amino acids, preferably in the range from 328 to 371 amino acids.

More specifically, the long-chain unspecific peroxidase may be selected from the group comprising or consisting of *Agrocybe aegerita* (syn. *Cyclocybe aegerita*) UPO (AaeUPO) and *Coprinopsis verticillata* UPO-II (CveUPO).

Typically, the short-chain unspecific peroxidase comprises a sequence of amino acids in the range from 200 to 299 amino acids, preferably in the range from 236 to 260 amino acids.

More specifically, the long-chain unspecific peroxidase may be selected from the group comprising or consisting of *Marasmius rotula* UPO (MroUPO), *Chaetomium globosum* UPO (CgIUPO) and *Collariella virescens* UPO (CviUPO).

The RNA polymerase used in the method according to the present invention is not especially limited. Preferably, the RNA polymerase is selected from the group comprising T3, T7 polymerase, and SP6 polymerase

The insect cells or mammalian cells used in the present invention are not especially limited. Principally, any insect cells or mammalian cells used in the prior art for preparing cell lysates are suitable.

Preferably, these insect cells or mammalian cells are capable to provide cell lysates containing microsomes, i.e. endogenous ER-derived membrane vesicles. Thus, in a preferred embodiment of the claimed method, the ER-derived membrane vesicles in the reaction medium originate from the same cell line as the cell lysate and are typically provided by and contained in said cell lysate.

Such microsomes allow protein translocation and posttranslational modifications of the target proteins such as N-glycosylations and were reported for several mammalian and insect cell based systems. Furthermore, the microsomal lumen is much more favorable for the formation of disulfide bonds compared to the bulk reaction medium outside the microsomes.

Specifically, the cells may be selected from the group which comprises *Spodoptera frugiperda* cells, CHO cells and K562 cells.

The preparation of cell lysates from the various eukaryotic cells mentioned above is well known in the art (e.g. Brödel, 2013 https://doi.org/10.1002/bit.25013) and, if desired, suitable modifications of known procedures can be effected by the skilled artisan.

According to the present invention, the respective cell lysate advantageously was supplemented with glutathione and glutathione-disulfide (GSH/GSSG) during lysate preparation in order to promote the formation of disulfide bonds. Herein, a cell lysate supplemented in this manner is also referred to as a redox-optimized cell lysate.

Redox optimization already during the lysate preparation was not an obvious step in the preparation of the insect or mammalian cell lysates used in the method of the present invention. Due to the supplementation with glutathione and glutathione-disulfide (GSH/GSSG) the cytosolic components are exposed to-non-natural redox conditions which might impair some activity of said components. This, e.g., could result in a less active translation apparatus and in a generally less active cell lysate leading to decreased protein yields.

Further, typically the addition of dithiothreitol (DTT) in the course of the lysate preparation was omitted according to the present invention. Since DTT conventionally is added in order to stabilize the respective enzymes and to protect the lysate components from oxidation, a lower activity of the resulting lysate and/or less storage stability could be expected.

However, advantageously no detrimental consequences of the redox optimization as effected herein could be observed by the present inventors.

The respective concentrations and ratios of the GSH/GSSG added are not especially critical.

For example, the respective concentrations may be in the mM range, i.e. in a concentration of 0.01 mM to 10 mM, preferably in a range from 0.05 mM to 5 mM or 5 mM to 10 mM, and the ratio of GSH/GSSG may vary from 1:1 to 1:30, preferably from 1:2 to 1:10 or 1:5.

In a closely related aspect, the present invention further relates to a reaction medium, in particular for preparing a soluble and functional peroxygenase in acell-free system, comprising at least the following components:
- a nucleic acid template coding for a fungal unspecific peroxygenase (UPO) classified as EC 111.2.1
- an RNApolymerase
   a eukaryotic cell lysate derived from insect cells or mammalian cells and supplemented with glutathione and glutathione-disulfide during lysate preparation wherein the cell lysate further comprises non-fungal membrane vesicles
- heme or a heme derivative, in particular a heme complex selected from the group comprising or consisting of hematin and hemin.

In a more specific embodiment of the invention, the reaction medium comprises hemin or hematin in a concentration in the range of from 1 µM to 100 µM, preferably from 15 µM to 20 µM.

A further aspect of the present invention relates to a method for screening and characterization of mutants or variants of unspecific peroxygenases classified as EC 1.11.2.1. which method comprises steps a)- b) of the method according to claim 1 and further at least step c) screening the functional peroxygenase obtained after step b) for a target property, such as substrate specificity or stereoselectivity.

More specifically, in said method step c) is effected by means of an assay selected from the group comprising:
- colorimetric assays of catalytic activity, e.g. ABTS/DMP/NBD-Assays, for identifying new active UPOs
- competitive colorimetric assays for qualitative or quantitative determination of the binding of a target substrate by inhibiting the turnover of a specific substrate, e.g. ABTS/DMP/NBD, particularly in order to screen for variants having an increase substrate specificity
- mass spectrometric analysis of the product of an UPO-catalyzed reaction in order to screen for UPO-variants having a desired substrate turnover.

### Brief description of the Figures

**Fig. 1** shows SDS-PAGE and autoradiography of cell-free synthesized AaeUPO and MroUPO.
   (A) Schematic drawing of fractionation; (B + C) Samples of the respective fractions were acetone precipitated and subsequently prepared for SDS-PAGE and analyzed through autoradiography.
**Fig. 2** shows the effect of hematin concentration in the reaction medium on the yield (expressed as relative activity) of UPOs MroUPO and AaeUPO, respectively.
**Fig. 3** shows the activity of cell-free synthesized AaeUPO and MroUPO. (A) Activity of cfAaeUPO and cfMroUPO determined through ATBS-Assay. (B) DMP-assay for various cfMroUPO constructs either containing no signal peptide or a melittin signal peptide (Mel-MroUPO).

The present invention is further illustrated by the following specific but non-limiting examples.

### EXAMPLE 1

### Synthesis and characterization of AaeUPO and MroUPO

An insect cell lysate derived from *Spodoptera frugiperda* cells, i.e. *Sf*21 cells, was utilized for cell-free synthesis of two exemplary peroxidases, AaeUPO and MroUPO. To achieve efficient translocation the sequences coding for the native signal peptide of AaeUPO and MroUPO were replaced by the melittin-signalpeptide, which was shown to enhance translocation as well as translation efficiency in Sf21 and CHO CFPS (Brödel et al. 2014). Furthermore, the lysate was supplemented with glutathione and glutathione-disulfide (GSH/GSSG) during lysate preparation to promote the formation of disulfide bonds. Utilizing this redox-optimized lysate both AaeUPO and MroUPO were synthesized. The translation mix (TM) was supplemented with ¹⁴C-leucine, which allowed visualization through autoradiography after SDS-PAGE.

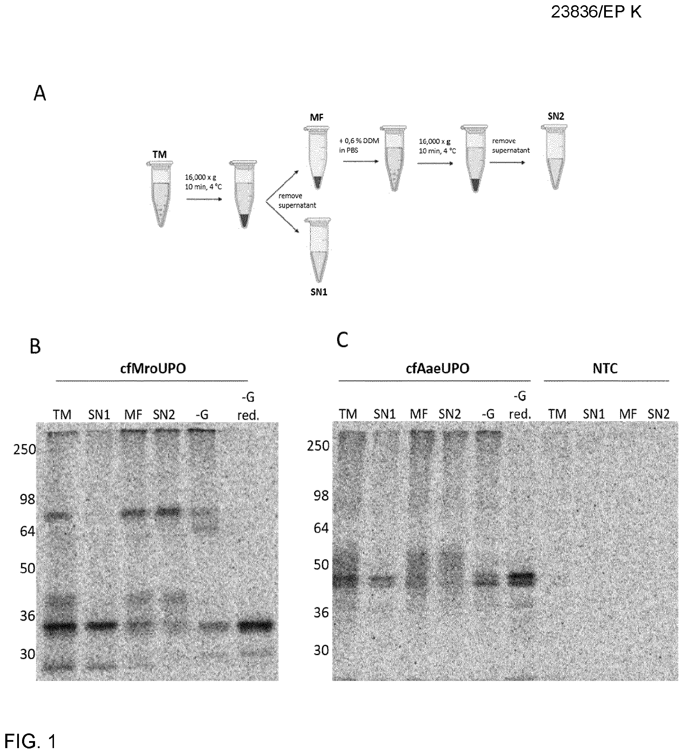

Fig. 1 shows SDS-PAGE and autoradiography of cell-free synthesized AaeUPO and MroUPO. (A) Schematic drawing of fractionation; (B + C) Samples of the respective fractions were acetone precipitated and subsequently prepared for SDS-PAGE and analyzed through autoradiography.

After synthesis the TM was fractionated through centrifugation, resulting in the supernatant 1 (SN1) containing all soluble components of the cell-free reaction and the pelleted microsomal fraction (MF) containing the microsomes including the translocated protein. Subsequently the microsomes were treated with 0.5 % CHAPS to release translocated protein from the microsomes lumen. An additionally centrifugation step was performed to separate soluble protein (SN2) from any remaining insoluble debris. For MroUPO a single band and for AaeUPO a double band at 34 kDa and 45 kDa, was detected respectively. Additionally, weaker bands are observed for both UPOs in the fractions TM, MF and SN2, which are no longer present after PNGase F digestion, indicating glycosylation. This is in accordance with their absence in the SN1, where no glycosylation should occur. In the fractions TM, MF and SN2 cfMroUPO displays another band about at twice the height as the main band, which is no longer visible under reducing conditions. This in accordance with the reported 3D-structure of MroUPO, that form dimers through disulfide bridges. The absence in the SN1 is not surprising, since disulfide bonds are not expected to form in a cytosolic environment. No distinct bands were observed in the no-template-control (NTC), which comprises a cell-free reaction without any added template, that was treated equally during all processing steps.

The activity of the UPOs produced were determined through conversion of known UPO substrates ABTS (2,2'-azino-bis(3-ethylbenzothiazoline-6-sulfonic acid)), DMP (2,6-Dimethoxyphenol) and NBD (5-nitro-1,3-benzodioxole). The conversion can be monitored colorimetrically.

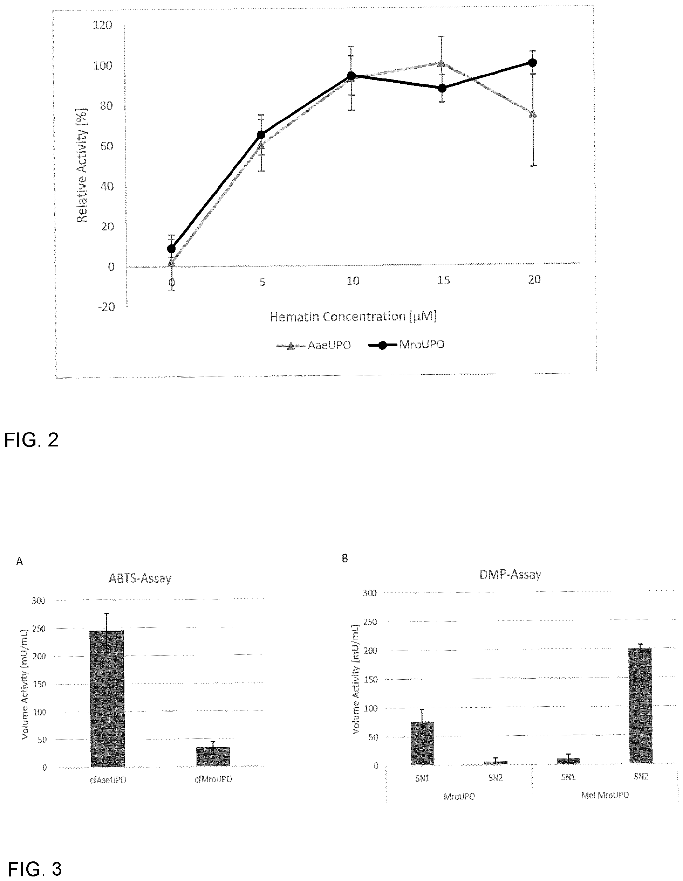

Fig.3 shows the activity of cell-free cfAaeUPO and cfMroUPO. (A) Activity of cfAaeUPO and cfMroUPO determined through ATBS-Assay. (B) DMP-assay for different cfMroUPO construct either containing no signal peptide or a melittin signal peptide (Mel-MroUPO). Different fractions of translation reactions were analyzed comprising either, the reaction supernatant SN1 or the soluble part of the microsomal fraction SN2.

ABTS was successfully converted by both UPOs obtained from the SN2 with volume activities of 244 mU/ml and 33 mU/ml for AaeUPO and MroUPO, respectively (Fig. 3 A) corresponding to specific activities of 116 U/mg and 14 U/mg. No template controls (NTC) were always included taking into account any background activity due to other endogenous proteins capable to oxidize ABTS.

The DMP assay showed pronounced activity of MroUPO but not for AaeUPO (Fig. 3 B). Though MroUPO was expected to exhibit a higher activity towards DMP than AaeUPO, the complete lack of detectable activity of AaeUPO towards DMP is somewhat surprising. In contrast to the ABTS-assay no clouding was observed adding SN1 to the DMP-assay buffer. MroUPO showed a volume activity of 200 mU/mL in the SN2 and a specific activity o 127 U/mg. The background activity exhibited in the NTC was much lower than for ABTS (2.3 mU/mL).

In further experiments, the contribution of protein translocation to the activity of cell-free synthesized UPO was investigated. Two MroUPO constructs were used for synthesis and subsequent activity assays, one without any signal-peptide and the other as described above with a melittin signal peptide (Mel). Without Mel the activity was only overserved in the reaction supernatant SN1 (76 mU/mL), whereas the addition of a signal peptide leads to activity in the SN2 (200 mU/ml). Mel-MroUPO in the SN2 exhibited a substantially higher specific activity of 127 U/mg than MroUPO without signal peptide in the SN1 with 29 U/mg. Interestingly, no activity was observed in the SN1 of Mel-MroUPO, although it shows a higher protein yield in said fraction. The higher protein yield for the Mel-construct is in accordance with previous reports about Mel enhancing the translation efficiency, as described above. The absence of any detectable activity in the SN1 of Mel-MroUPO might be due to the uncleaved signal peptide. Deviations from the native sequence of the N-terminus were shown previously to have an negative effect on activity (Püllmann et al. 2021). These results underline the importance of translocation for protein activity. The effects of the translocation can be traced back on mainly three factors, formation of disulfide bonds, N-glycosylation and protein folding in general.

### Detailed experimental procedures

### Design of DNA Templates

The templates for the Sf21 based synthesis of AaeUPO and MroUPO were ordered as plasmids from Biocat GmbH (Heidelberg). The construct design was based on the design reported earlier (Brödel et al., 2013). The constructs were equipped with a T7-Promotor. An IRES from the Cricket-paralysis virus was utilized to enhance cap-independent translation initiation. The native signal peptide was removed and constructs with and without melittin signal peptide were prepared. As plasmid backbone pUC57-1.8k was used.

### Lysate preparation

Sf21 lysates preparation and coupled CFPS in general have been described previously and explained in (Brödel et al., 2013; Quast et al., 2015). Minor changes were made during lysate preparation. In all buffers used during the lysate preparation DTT was omitted and GSH/GSSG in the mM range in a ratio of 1:5 were added. Coupled transcription-translation reactions were performed in a batch mode format. Protein synthesis was mainly performed at optimal reaction temperature in a thermomixer (Thermomixer comfort, Eppendorf, Hamburg, Germany) with gentle shaking at 500 rpm for 2h. Reaction volumes of 50µl were composed of 40% (v/v) Sf21 cell lysate, 100 mM of each canonical amino acids, nucleoside triphosphates (1.75 mM ATP, 0.30 mM CTP, 0.30 mM GTP, and 0.30 mM UTP), 60 nM vector DNA, and 1 U/ml T7 RNA-polymerase (Agilent, Waldbronn, Germany), 15 µM hematin (if not stated else). To monitor protein quality and quantity, reaction mixtures were supplemented with ¹⁴C-labeled leucine (specific radioactivity 75.0 dpm/pmol). No template controls (NTC) were prepared in the same way as the samples with the exception of the DNA template which was replaced by RNase-free water.

### Fractionation

After incubation the translation mix (TM) was centrifuged at 16,000xg and 4 °C for 10 min. The resulting supernatant was collected (SN1) and the microsome harboring pellet was resuspended in phosphate buffered saline (PBS) containing 0.5 % 3-[(3-cholamidopropyl)dimethylammonio]-1-propanesulfonate (CHAPS). The resuspended pellet was incubated at 21 °C and 750 rpm for 45 min, to release translocated proteins out of the microsomes. Subsequently, the samples were centrifuged again, and the supernatant (SN2) was collected.

### Quantitative analysis through Liquid scintillation counting

Total protein yields of cell-free synthesized, ¹⁴C-labeled protein were determined by hot trichloroacetic acid (TCA) precipitation and subsequent liquid scintillation counting as previously described. In short, 3 µl of the analyzed fraction were mixed with 10 % TCA (Carl Roth GmbH) with 2 % casein hydrolysate (Carl Roth GmbH) and incubated in a water bath at 80 °C for 15 min, followed by a 30 min incubation step on ice. The TCA-solution was then transferred onto membrane filters (VWR) using a vacuum filtration device (Hoefer). The filters were washed with 5 % acetone and dried with acetone. The dry filters were transferred into scintillation vials (Sarstedt AG & Co KG) and incubated in 3 ml scintillation cocktail for 1 h. Subsequently, the samples were measured using the Hidex 600 SL (Hidex). Protein yields were calculated based on the counted disintegrations per minute, the specific activity of leucine in the cell free reaction, the molecular weight and the number of leucines in the protein.

### SDS-PAGE analysis

For qualitative analysis 3 µl of the analyzed fraction was mixed with 47 µl water and precipitated in cold acetone (Carl Roth GmbH) for at least 15 min. After centrifugation at 16,000 g at 4 °C for 10 min the supernatant was removed and the precipitate dried 30 min at 45°C. Precipitate was resolubilized through the addition of NuPage loading buffer with or without 50 mM DTT. Sodium dodecyl sulfate polyacrylamide gels with 10 % acrylamide were prepared using SureCast resolving and stacking buffer (Thermo Fisher Scientific). The SeeBlue Pre-Stained marker (Thermo Fisher) was used as standard. Gels were run at 150 V for 60 min and subsequently dried at 70 C (Unigeldryer 3545D, Uniequip, Planegg). Dry gels were placed on a phosphor screen (GE Healthcare). After 1 to 2 days the phosphor screen was scanned using a Typhoon Trio + variable mode imager (GE Healthcare) to visualize labeled proteins.

### Deglycosylation Assay

Protein N-glycosylation was investigated through PNGase F (peptide N-glycosidase F, NEB) digestion according to the manufacturers protocol. Of the fraction of interest 5 µl were used for PNGase F digestion. After incubation the digested samples were acetone precipitated and analyzed through SDS-PAGE and autoradiography as described above.

### Activity Assays

Activity of UPO were analyzed through the conversion of 2,2'-azino-bis(3-ethylbenzothiazoline-6-sulfonic acid) (ABTS) to the ABTS radical cation (ε₄₁₈ = 36000 mM⁻¹ cm⁻¹), two 2,6-diemthoyphenol (DMP) to coerulignone (ε469 = 49600 mM⁻¹ cm⁻¹) and 5-nitro-1,3-benzodioxole (NBD) to 4-nitrocatechol (ε425 = 9700 mM⁻¹ cm⁻¹). For the activity assay 10 µl to 20 µl sample were added to 80 µl to 70 µl assay buffer. The assay buffer compositions were as follows. **ABTS:** Mcllvaine buffer pH 4.5, 0.3 mM ABTS, 10 mM GSSG; **DMP:** Potassium phosphate buffer pH 6 (MroUPO) or pH 7 (AaeUPO), 6 mM DMP; **NBD:** Mcllvaine buffer pH7, 5 mM NBD. The reactions were started by adding 10 µl 0,06 % H₂O₂. Extinction measurements were performed using a microplate reader (Mithras LB 943 Multimode Plate Reade, Berthold Technologies GmbH & Co. KG).

### EXAMPLE 2

### Effect of hematin concentration in the reaction medium on the yield of MroUPO and AaeUPO

Until now no hemo-protein was produced in an insect-based cell-free system for protein synthesis. To assess the influence of heme addition on the protein synthesis, different heme concentrations were applied. The heme was supplemented as hematin dissolved in 5.3 mM NaOH. The activity of the produced UPO were monitored through DMP and ABTS-assays for MroUPO and AaeUPO respectively.

Fig. 2 shows the effect of hematin concentration on the yield of active UPO, expressed as normalized activity of MroUPO and AaeUPO synthesized at different hematin concentrations determined through DMP and ABTS-Assay, respectively. Two different assays were chosen to maximize the readout, since both UPOs are known to display a different activity to each substrate. For both UPOs no activity was observed without hematin supplementation indicating that no or at least not sufficient heme is present in the native lysate. UPO activity is increasing with hematin concentrations up to 10 µM. For AaeUPO the maximum activity is reached at 15 µM hematin, with a drastic decreasing activity at higher concentrations. MroUPO however, shows a higher tolerance towards hematin, with a maximum at 20 µM hematin.

Based on these data and for the sake of simplicity and comparability all further reactions were performed with 15 µM hematin.

## Claims

1. A method for preparing a soluble and functional peroxygenase in cell-free systems comprising at least the following steps:
a) providing a reaction medium comprising at least the following components:
- a nucleic acid template coding for a fungal unspecific peroxygenase (UPO) classified as EC 111.2.1
- an RNApolymerase
- a eukaryotic cell lysate derived from insect cells or mammalian cells and supplemented with glutathione and glutathione-disulfide during lysate preparation wherein the cell lysate further comprises non-fungal membrane vesicles
- heme or a heme derivative, in particular a heme complex selected from the group comprising or consisting of hematin and hemin;
b) performing a cell-free reaction to synthesize the peroxygenase protein.

2. The method according to claim 1, wherein the nucleic acid template coding for a fungal unspecific peroxygenase is a DNA template.

3. The method according to claim 1 or 2, wherein nucleic acid template further comprises a nucleic sequence coding for a melittin signal peptide.

4. The method according to anyone of claims 1 to 3, wherein membrane vesicles originate from the same cell line as the cell lysate.

5. The method according to anyone of claims 1 to 4, wherein the insect cells are *Spodoptera frugiperdo* cells or the mammalian cells are selected from CHO and K562 cells.

6. The method according to any one of claims 1 to 5, wherein the cell-free translation reaction is effected at a temperature in the range from 21 to 33 °C.

7. The method according to any one of claims 1 to 6, wherein the fungal unspecific peroxidase is a long-chain unspecific peroxidase comprising a sequence of amino acids in the range from 300 to 400 amino acids, preferably from 328 to 371 amino acids.

8. The method according to claim 7, wherein the fungal unspecific peroxidase is selected from the group comprising *Agrocybe aegerita* UPO (AaeUPO) and *Coprinopsis verticillata* UPO-II (CveUPO).

9. The method according to any one of claims 1 to 6, wherein the fungal unspecific peroxidase is a short-chain unspecific peroxidase comprising a sequence of amino acids in the range from 200 to 300 amino acids, preferably from 236 to 260 amino acids.

10. The method according to claim 9, wherein the fungal unspecific peroxidase is selected from the group comprising *Marasmius rotula* UPO (MroUPO), *Chaetomium globosum* UPO (CgIUPO) and *Collariella virescens* UPO (CviUPO).

11. The method according to any one of claims 1-10, wherein the RNA polymerase is selected from the group comprising T3, T7 polymerase, and SP6 polymerase.

12. A reaction medium for preparing a soluble and functional peroxygenase in a cell-free system comprising at least the following components:
- a nucleic acid template coding for a fungal unspecific peroxygenase (UPO) classified as EC 111.2.1
- an RNApolymerase
- a eukaryotic cell lysate derived from insect cells or mammalian cells and supplemented with glutathione and glutathione-disulfide during lysate preparation wherein the cell lysate further comprises non-fungal membrane vesicles
- heme or a heme derivative, in particular a heme complex selected from the group comprising or consisting of hematin and hemin.

13. The reaction medium according to claim 12 or the method according to any one of claims 1-11, wherein the reaction medium comprises the heme or heme derivative in a concentration in the range of from 1 µM to 100 µM, preferably from 15 µM to 20 µM.

14. A method for screening and characterization of mutants or variants of unspecific peroxygenases classified as EC 1.11.2.1., which method comprises steps a)- b) of the method according to claim 1 and further at least
step c) screening the functional peroxygenase obtained after step b) for a target property, such as substrate specificity or stereoselectivity.

15. The method according to claim 14, wherein step c) is effected by means of an assay selected from the group comprising:
- colorimetric assays of catalytic activity, e.g. ABTS/DMP/NBD-Assays, for identifying new active UPOs
- competitive colorimetric Assays for qualitative or quantitative determination of the binding of a target substrate by inhibiting the turnover of a specific substrate, e.g. ABTS/DMP/NBD, particularly in order to screen for variants having an increase substrate specificity
- mass spectrometric analysis of the product of an UPO-catalyzed reaction in order to screen for UPO-variants having a desired substrate turnover.

## Patentansprüche

1. Verfahren zur Herstellung einer löslichen und funktionellen Peroxygenase in zellfreien Systemen, umfassend mindestens die folgenden Schritte:
a) Bereitstellen eines Reaktionsmediums, das mindestens die folgenden Bestandteile umfasst:
- ein Nukleinsäuretemplat, das für eine unspezifische Pilz-Peroxygenase (UPO) kodiert, die als EC 1.11.2.1 klassifiziert ist,
- eine RNA-Polymerase,
- ein eukaryotisches Zelllysat, das von Insektenzellen oder Säugerzellen stammt und mit Glutathion und Glutathiondisulfid während der Lysatherstellung ergänzt wird, wobei das Zelllysat ferner Nicht-Pilz-Membranvesikel umfasst,
- Häm oder ein Hämderivat, insbesondere einen Hämkomplex, ausgewählt aus der Gruppe, umfassend oder bestehend aus Hämatin und Hämin;
b) Durchführen einer zellfreien Reaktion zum Synthetisieren des Peroxygenaseproteins.

2. Verfahren nach Anspruch 1, wobei das Nukleinsäuretemplat, das für eine unspezifische Pilz-Peroxygenase kodiert, ein DNA-Templat ist.

3. Verfahren nach Anspruch 1 oder 2, wobei das Nukleinsäuretemplat ferner eine Nukleinsäuresequenz umfasst, die für ein Melittin-Signalpeptid kodiert.

4. Verfahren nach einem der Ansprüche 1 bis 3, wobei die Membranvesikel von der gleichen Zelllinie wie das Zelllysat stammen.

5. Verfahren nach einem der Ansprüche 1 bis 4, wobei die Insektenzellen *Spodoptera frugiperda*-Zellen sind oder die Säugerzellen aus CHO- und K562-Zellen ausgewählt sind.

6. Verfahren nach einem der Ansprüche 1 bis 5, wobei die zellfreie Translationsreaktion bei einer Temperatur im Bereich von 21 bis 33 °C bewirkt wird.

7. Verfahren nach einem der Ansprüche 1 bis 6, wobei die unspezifische Pilz-Peroxidase eine langkettige unspezifische Peroxidase ist, die eine Sequenz von Aminosäuren im Bereich von 300 bis 400 Aminosäuren, vorzugsweise von 328 bis 371 Aminosäuren, umfasst.

8. Verfahren nach Anspruch 7, wobei die unspezifische Pilz-Peroxidase aus der Gruppe, umfassend *Agrocybe aegerita* UPO (AaeUPO) und *Coprinopsis verticillata* UPO-II (CveUPO), ausgewählt ist.

9. Verfahren nach einem der Ansprüche 1 bis 6, wobei die unspezifische Pilz-Peroxidase eine kurzkettige unspezifische Peroxidase ist, die eine Sequenz von Aminosäuren im Bereich von 200 bis 300 Aminosäuren, vorzugsweise von 236 bis 260 Aminosäuren, umfasst.

10. Verfahren nach Anspruch 9, wobei die unspezifische Pilz-Peroxidase aus der Gruppe, umfassend *Marasmius rotula* UPO (MroUPO), *Chaetomium globosum* UPO (CgIUPO) und *Collariella virescens* UPO (CviUPO), ausgewählt ist.

11. Verfahren nach einem der Ansprüche 1-10, wobei die RNA-Polymerase aus der Gruppe, umfassend T3-, T7-Polymerase und SP6-Polymerase, ausgewählt ist.

12. Reaktionsmedium zur Herstellung einer löslichen und funktionellen Peroxygenase in einem zellfreien System, das mindestens die folgenden Bestandteile umfasst:
- ein Nukleinsäuretemplat, das für eine unspezifische Pilz-Peroxygenase (UPO) kodiert, die als EC 1.11.2.1 klassifiziert ist,
- eine RNA-Polymerase,
- ein eukaryotisches Zelllysat, das von Insektenzellen oder Säugerzellen stammt und mit Glutathion und Glutathiondisulfid während der Lysatherstellung ergänzt wird, wobei das Zelllysat ferner Nicht-Pilz-Membranvesikel umfasst,
- Häm oder ein Hämderivat, insbesondere einen Hämkomplex, ausgewählt aus der Gruppe, umfassend oder bestehend aus Hämatin und Hämin.

13. Reaktionsmedium nach Anspruch 12 oder Verfahren nach einem der Ansprüche 1-11, wobei das Reaktionsmedium das Häm oder das Hämderivat in einer Konzentration in dem Bereich von 1 µM bis 100 µM, vorzugsweise von 15 µM bis 20 µM, umfasst.

14. Verfahren zum Screenen und Charakterisieren von Mutanten oder Varianten von unspezifischen Peroxygenasen, die als EC 1.11.2.1 klassifiziert sind, wobei das Verfahren die Schritte a) - b) des Verfahrens nach Anspruch 1 und ferner mindestens Schritt c) Screenen der funktionellen Peroxygenase, die nach dem Schritt b) erhalten worden ist, bezüglich einer Zieleigenschaft, wie z.B. einer Substratspezifität oder -stereoselektivität, umfasst.

15. Verfahren nach Anspruch 14, wobei der Schritt c) mittels eines Assays bewirkt wird, der aus der Gruppe, umfassend:
- kolorimetrische Assays der katalytischen Aktivität, z.B. ABTS/DMP/NBD-Assays, zum Identifizieren neuer aktiver UPOs,
- kompetitive kolorimetrische Assays zur qualitativen oder quantitativen Bestimmung des Bindens eines Zielsubstrats durch Hemmen des Umsatzes eines spezifischen Substrats, z.B. ABTS/DMP/NBD, insbesondere zum Screenen bezüglich Varianten mit einer erhöhten Substratspezifität,
- massenspektrometrische Analyse des Produkts einer UPO-katalysierten Reaktion zum Screenen bezüglich UPO-Varianten mit einem gewünschten Substratumsatz, ausgewählt ist.

## Revendications

1. Procédé de préparation d'une peroxygénase soluble et fonctionnelle dans des systèmes acellulaires comprenant au moins les étapes suivantes :
a) la fourniture d'un milieu réactionnel comprenant au moins les composants suivants :
- une matrice d'acide nucléique codant pour une peroxygénase fongique non spécifique (UPO) classée EC 111.2.1
- une ARN polymérase
- un lysat de cellules eucaryotes dérivé de cellules d'insecte ou de cellules de mammifère et complété avec du glutathion et du glutathion-disulfure pendant la préparation du lysat, dans lequel le lysat de cellules comprend en outre des vésicules membranaires non fongiques
- un hème ou un dérivé d'hème, en particulier un complexe d'hème sélectionné dans le groupe comprenant ou consistant en l'hématine et l'hémine ;
b) la réalisation d'une réaction acellulaire pour synthétiser la protéine peroxygénase.

2. Procédé selon la revendication 1, dans lequel la matrice d'acide nucléique codant pour une peroxygénase fongique non spécifique est une matrice d'ADN.

3. Procédé selon la revendication 1 ou 2, dans lequel la matrice d'acide nucléique comprend en outre une séquence nucléique codant pour un peptide signal de mélittine.

4. Procédé selon l'une quelconque des revendications 1 à 3, dans lequel les vésicules membranaires proviennent de la même lignée cellulaire que le lysat de cellules.

5. Procédé selon l'une quelconque des revendications 1 à 4, dans lequel les cellules d'insecte sont des cellules de *Spodoptera frugiperda* ou les cellules de mammifère sont sélectionnées parmi les cellules CHO et K562.

6. Procédé selon l'une quelconque des revendications 1 à 5, dans lequel la réaction de traduction acellulaire est effectuée à une température dans la plage de 21 à 33 °C.

7. Procédé selon l'une quelconque des revendications 1 à 6, dans lequel la peroxydase fongique non spécifique est une peroxydase non spécifique à chaîne longue comprenant une séquence d'acides aminés dans la plage de 300 à 400 acides aminés, de préférence de 328 à 371 acides aminés.

8. Procédé selon la revendication 7, dans lequel la peroxydase fongique non spécifique est sélectionnée dans le groupe comprenant l'UPO de *Agrocybe aegerita* (AaeUPO) et l'UPO-II de *Coprinopsis verticillata* (CveUPO).

9. Procédé selon l'une quelconque des revendications 1 à 6, dans lequel la peroxydase fongique non spécifique est une peroxydase non spécifique à chaîne courte comprenant une séquence d'acides aminés dans la plage de 200 à 300 acides aminés, de préférence de 236 à 260 acides aminés.

10. Procédé selon la revendication 9, dans lequel la peroxydase fongique non spécifique est sélectionnée dans le groupe comprenant l'UPO de *Marasmius rotula* (MroUPO), l'UPO de *Chaetomium globosum* (CglUPO) et l'UPO de *Collariella virescens* (CviUPO).

11. Procédé selon l'une quelconque des revendications 1 à 10, dans lequel l'ARN polymérase est sélectionnée dans le groupe comprenant la T3, la polymérase T7, et la polymérase SP6.

12. Milieu réactionnel pour la préparation d'une peroxygénase soluble et fonctionnelle dans un système acellulaire comprenant au moins les composants suivants :
- une matrice d'acide nucléique codant pour une peroxygénase fongique non spécifique (UPO) classée EC 111.2.1
- une ARN polymérase
- un lysat de cellules eucaryotes dérivé de cellules d'insecte ou de cellules de mammifère et complété avec du glutathion et du glutathion-disulfure pendant la préparation du lysat, dans lequel le lysat de cellules comprend en outre des vésicules membranaires non fongiques
- un hème ou un dérivé d'hème, en particulier un complexe d'hème sélectionné dans le groupe comprenant ou consistant en l'hématine et l'hémine.

13. Milieu réactionnel selon la revendication 12 ou procédé selon l'une quelconque des revendications 1 à 11, dans lequel le milieu réactionnel comprend l'hème ou le dérivé d'hème à une concentration dans la plage de 1 µM à 100 µM, de préférence de 15 µM à 20 µM.

14. Procédé de criblage et de caractérisation de mutants ou de variants de peroxygénases non spécifiques classées EC 1.11.2.1., lequel procédé comprend les étapes a) et b) du procédé selon la revendication 1 et en outre au moins
l'étape c) de criblage de la peroxygénase fonctionnelle obtenue après l'étape b) pour une propriété cible, telle qu'une spécificité pour un substrat ou une stéréosélectivité.

15. Procédé selon la revendication 14, dans lequel l'étape c) est effectuée au moyen d'un essai sélectionné dans le groupe comprenant :
- les dosages colorimétriques d'activité catalytique, par exemple les dosages ABTS/DMP/NBD, pour identifier de nouvelles UPO actives
- les dosages colorimétriques compétitifs pour la détermination qualitative ou quantitative de la liaison d'un substrat cible en inhibant le renouvellement d'un substrat spécifique, par exemple ABTS/DMP/NBD, en particulier afin de rechercher des variants présentant une spécificité accrue pour un substrat
- une analyse par spectrométrie de masse du produit d'une réaction catalysée par UPO afin de rechercher des variants d'UPO présentant un renouvellement de substrat souhaité.
